**Europäisches Patentamt**

(19) **European Patent Office**

**Office européen des brevets**

(11) Numéro de publication: **0 091 358**
**B1**

# (12) FASCICULE DE BREVET EUROPEEN

(45) Date de publication du fascicule du brevet:
**22.05.85**

(21) Numéro de dépôt: **83400645.4**

(22) Date de dépôt: **29.03.83**

(51) Int. Cl.⁴: **C 07 C 17/10, C 07 C 23/10**

(54) **Procédé et appareil pour la production photochimique d'halogéno alcanes et cyclo-alcanes.**

(30) Priorité: **07.04.82 FR 8206031**

(43) Date de publication de la demande:
**12.10.83 Bulletin 83/41**

(45) Mention de la délivrance du brevet:
**22.05.85 Bulletin 85/21**

(84) Etats contractants désignés:
**BE CH DE GB IT LI NL**

(56) Documents cités:
**FR - A - 1 254 403**
**US - A - 1 954 438**
**US - A - 3 378 476**

(73) Titulaire: **SOCIETE NATIONALE ELF AQUITAINE (PRODUCTION), Tour Aquitaine, F-92400 Courbevoie (FR)**

(72) Inventeur: **Ollivier, Jean, Croix de Buzy, F-64260 Arudy (FR)**

(74) Mandataire: **Kohn, Armand, 5 Avenue Foch, F-92380 Garches (FR)**

## Description

L'invention concerne la fabrication d'halogénoalcanes et d'halogénocycloalcanes, en phase liquide, par l'action du chlore sur l'hydrocarbure correspondant. Elle apporte un procédé perfectionné, ainsi qu'un appareil pour la réalisation de ce procédé. L'invention s'applique très avantageusement à la production de chlorocycloalcanes, en particulier du monochlorure de cyclohexyle.

Vu l'importance des halogénoalcanes et des cycloalcanes, notamment du chlorure de cyclohexyle, comme produits intermédiaires dans la production de différents composés utiles, la préparation de ces corps a donné lieu à un certain nombre de réalisations industrielles. Parmi les procédés existants, le plus pratique est celui qui consiste à faire passer une solution de chlore dans du cyclohexane, dans un réacteur, dans lequel est placé un émetteur de lumière ultraviolette; le monochlorocyclohexane ainsi formé est ensuite séparé par distillation du cyclohexane restant. Cependant, dans la pratique industrielle de cette méthode, on se heurte à l'inconvénient d'une chloration trop poussée, conduisant à des dérivés polychlorés du cyclohexane, abaissant la sélectivité de la réaction en monochlorocyclohexane désiré. On perd ainsi des quantités de l'ordre de plus de 10% de cyclohexane sous forme de composés polychlorés, comme on peut le voir par exemple au tableau de la page 2 du BF N° 1254403 et au tableau 1 du USP N° 3494844. Cet inconvénient était attribué, par certains chercheurs, à la réaction du chlore avec le cyclohexane dans l'obscurité avant que le mélange réactionnel n'arrive dans la zone où a lieu l'émission de l'ultraviolet. Dans cette optique, on a conçu un procédé qui consiste à introduire une faible proportion d'oxygène dans le milieu réactionnel, afin d'inhiber la chloration dans l'obscurité; par ce moyen, on devait arriver à ce que la réaction commence seulement lorsque le mélange est irradié par l'utraviolet. Mais l'introduction de l'oxygène dans le milieu réactionnel comporte des risques contre lesquels on est obligé de prendre des précautions assez strictes, tant à cause des dangers que présente l'introduction de l'oxygène dans un hydrocarbure qu'à cause de l'action inhibitrice de l'oxygène sur la réaction photochimique elle-même. Il est donc nécessaire de contrôler rigoureusement l'entrée de l'oxygène dans le circuit d'alimentation, de façon que sa teneur ne soit que de l'ordre de 5 à 10 ppm, compte tenu de l'oxygène déjà normalement présent dans les réactifs. D'autre part, la puissance lumineuse à fournir au réacteur doit être accrue à cause de l'action inhibitrice susmentionnée. Il est à noter enfin que les peroxydes formés à partir de l'oxygène présent conduisent à la formation d'impuretés, notamment de cyclohexanol.

La présente invention permet d'éviter les inconvénients précités et de conduire à des sélectivités très élevées de plus de 96% en monochlorocyclohexane par rapport au cyclohexane transformé. Elle rend possible la conduite très régulière de la fabrication, sans danger et sans formation de quantités notables de sous-produits.

L'invention est fondée sur la découverte assez surprenante faite par les titulaires en ce qui concerne la propagation de la réaction d'halogénation dans l'obscurité, qui précède l'irradiation par l'ultraviolet; on est en effet arrivé à la conclusion étonnante que cette réaction dans les parties obscures, en amont du réacteur, est due à la propagation de la réaction initiée dans le réacteur photochimique. Effectivement, on a trouvé qu'en disposant un inhibiteur de chloration à l'entrée du réacteur la chloration ne se produit plus dans les appareils et les canalisations en amont du réacteur où a lieu la dissolution du chlore dans le cyclohexane.

Le procédé suivant l'invention est donc caractérisé en ce qu'une substance solide, insoluble dans le milieu réactionnel, inhibitrice de l'halogénation, est placée dans la région d'entrée du réacteur photochimique.

Plus particulièrement, le procédé suivant l'invention consiste à placer, à l'intérieur d'une région d'entrée du réacteur, un métal susceptible d'inhiber la chloration de l'hydrocarbure. Suivant un trait préféré de l'invention, le métal inhibiteur est choisi dans le groupe VB de la classification périodique des éléments, c'est-à-dire qu'elle est constituée par un ou plusieurs des métaux suivants: vanadium, niobium et tantale. Le tantale est particulièrement indiqué du fait de sa haute réactivité vis-à-vis des radicaux et atomes, et de son inertie chimique vis-à-vis du milieu réactionnel, riche en acide chlorhydrique.

De préférence, un tel inhibiteur est également placé dans la région de sortie du réacteur, ce qui évite la poursuite de la réaction en dehors du réacteur dans le cas où l'effluent contiendrait du chlore.

Bien que l'inhibition suivant l'invention soit un phénomène de type catalytique, n'exigeant qu'une faible quantité de métal inhibiteur, il est préférable que la surface de contact de l'inhibiteur avec le liquide entrant dans le réacteur soit suffisante pour que l'inhibition puisse se produire en tous les points de la veine de liquide coulant vers le réacteur. Il est donc recommandable que la surface offerte par le métal dans les régions d'entrée et de sortie du réacteur photochimique soit d'environ 100 à 10 000 cm² par centimètre carré de section de la veine liquide traversant ces régions. De toute manière, les poids et volume de métal inhibiteur employé sont incomparablement plus faibles que ceux des matériaux de construction du réacteur et des canalisations concernées.

L'effet inhibiteur de propagation des radicaux libres, par l'application de la présente invention, peut être obtenu de différentes manières. On peut notamment recouvrir les parois intérieures des conduites d'entrée et de sortie du réacteur photochimique, sur une certaine longueur, avec un chemisage de métal inhibiteur. Cependant, pour assurer un meilleur contact entre le liquide réactionnel et ce métal, il est préférable de disposer celui-ci sous une forme présentant une grande surface, transversalement au courant liquide. Ainsi le métal peut-il être placé sous la forme d'une ou de plusieurs plaques perforées ou grilles, perpendiculairement au trajet du liquide, dans les conduites en question. La conformation du type mélangeur statique est particulièrement recommandée. Comme les métaux inhibiteurs sont assez coûteux, ces plaques, très minces, ou grilles sont supportées par des plaques perforées ou grilles plus épaisses en matériau résistant, notamment céramique, verre ou métal inattaquable par le milieu réactionnel.

Comme il y a intérêt à utiliser l'inhibiteur, en particulier le vanadium, le niobium ou le tantale, sous une forme présentant une surface aussi grande que possible pour un minimum de poids, le moyen préféré consiste à prendre ces métaux en fins filaments ou en tournures de faible épaisseur. Ainsi est-il recommandable de se servir des filaments ayant une section d'environ 0,05 à 1 mm, et de préférence de 0,1 à 0,5 mm de diamètre, ou de tournures présentant une épaisseur de ce même ordre de grandeur. Dans ce cas, il convient de placer les filaments ou/et les tournures dans une boîte ou raccord délimité de part et d'autre par des grilles ou plaques perforées en matériau résistant, pour retenir l'inhibiteur en place, empêchant son entraînement par le courant liquide des réactifs traités.

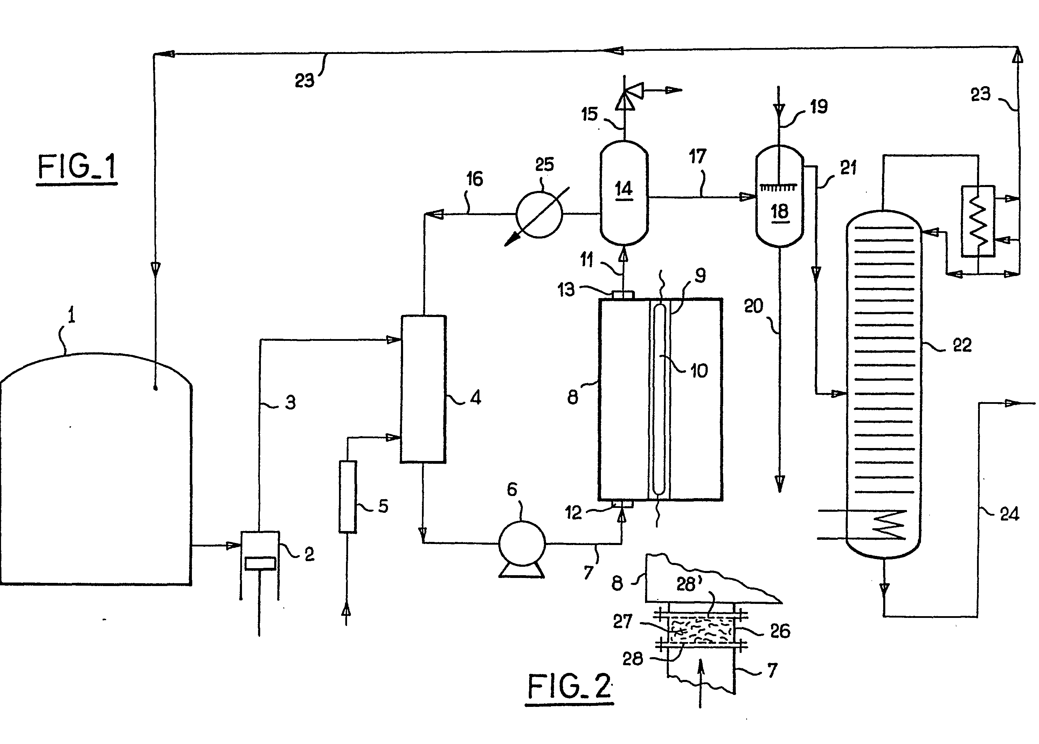

Cette forme d'exécution préférée est illustrée schématiquement sur la fig. 2 dont la description est donnée plus loin, à la suite de celle d'une installation (fig. 1) comprenant un garnissage de métal inhibiteur suivant l'invention.

Comme déjà indiqué plus haut, la surface offerte par le métal inhibiteur est de l'ordre de 100 à 10 000 cm$^2$ par centimètre carré de section de l'entrée et de la sortie du réacteur photochimique. Il est à remarquer cependant que la surface nécessaire dépend également de la vitesse du liquide traité. En effet, plus cette vitesse est grande à l'entrée du réacteur, moins grands sont les risques de propagation de radicaux libres vers l'amont du réacteur; inversement, plus la vitesse est grande, plus il y a des dangers d'entraîner de tels radicaux propagateurs de la réaction à la sortie du réacteur photochimique. Par conséquent, aux vitesses faibles du liquide doivent correspondre les surfaces d'inhibition supérieures des limites sus-indiquées, à l'entrée du réacteur; par contre, ce sont les limites inférieures de surface qui suffisent à la sortie, lorsque la vitesse est faible. C'est l'inverse qui est valable pour les fortes vitesses d'écoulement du liquide.

Pratiquement, les valeurs de surface sus-indiquées conviennent pour des vitesses d'environ 0,05 à 5 m/s à l'entrée du réacteur.

L'application d'un inhibiteur à l'entrée du réacteur photochimique suivant l'invention présente une grande importance; par contre, il est possible d'utiliser des surfaces d'inhibiteur faibles, ou même de les supprimer complètement à la sortie dans la forme d'exécution de l'invention qui consiste à régler la proportion d'halogène de telle manière qu'il soit entièrement combiné pendant le passage du liquide réactionnel par la région soumise à l'ultraviolet. Dans ces conditions, comme il n'y a plus d'halogène à la sortie du réacteur, la réaction ne se poursuit pas au-delà de celui-ci.

Comme il peut cependant y avoir des radicaux libres créés accidentellement dans le circuit de recyclage, ce qui initierait la réaction dans le liquide recyclé dès l'injection du chlore, la précaution suivant l'invention reste néanmoins valable et utile; autrement dit, il y a toujours intérêt à utiliser un garnissage d'inhibiteur à la sortie du réacteur.

En ce qui concerne les conditions générales de la synthèse suivant l'invention, elles sont semblables à celles de la technique antérieure: la température peut aller avantageusement de 0 à 50° C, et de préférence de 10 à 30° C; on peut employer des UV de longueurs d'onde de 250 à 400 nm, et plus particulièrement de 350 nm; le temps de séjour du mélange réactionnel dans le réacteur photochimique est de l'ordre de 1 à 300 s, et plus particulièrement de 3 à 150 s.

Comme dans la technique connue, le procédé suivant l'invention est réalisé de préférence avec un large excès d'alcane ou de cycloalcane par rapport à l'halogène. Ainsi, dans la préparation du chlorure de cyclohexyle, il est avantageux d'avoir au moins 5 mol de cyclohexane par mol de chlore, cette proportion pouvant d'ailleurs aller jusqu'à 200 pour 1.

L'invention, illustrée par les exemples donnés plus loin, peut être réalisée dans une installation représentée sur les figures annexées.

La fig. 1 est un schéma général d'une installation pour la chloration d'un alcane ou d'un cycloalcane liquide.

La fig. 2 représente en coupe schématique axiale la région du fond d'un réacteur photochimique avec une fraction de la conduite d'entrée des réactifs dans ce fond.

Sur le dessin, 1 désigne un réservoir de stockage de cyclohexane, 2 une pompe branchée au bas de ce réservoir, 3 une conduite pour la transfert du cyclohexane dans le mélangeur 4. Le chlore, qui arrive d'un réservoir non représenté à la pompe 5, est introduit dans la partie inférieure du mélangeur 4; la solution de chlore dans le cyclohexane est soutirée du mélangeur 4 par la pompe 6 pour être injectée par la canalisation 7 dans le bas du réacteur 8. Ce dernier contient un tube axial 9 en quartz, dans lequel se trouve une lampe à UV 10; une tubulure 11 constitue la sortie du haut du réacteur 8.

Conformément à la caractéristique originale de l'invention, à l'entrée du réacteur 8, la canalisation 7 se termine par un raccord 12 contenant de la tournure de tantale. Un raccord semblable 13 se trouve à la jonction de la tubulure de sortie 11 avec le réacteur 8; ce raccord 13 contient également de la tournure de tantale.

La tubulure 11 relie le réacteur avec le bas d'un séparateur 14 d'où les gaz sont évacués par un tuyau supérieur 15. De la partie inférieure du séparateur 14 part une conduite de recyclage 16 qui ramène la majeure partie du milieu réactionnel en haut du mélangeur 4; sur la conduite 16 est branché un échangeur de chaleur 25 permettant de refroidir à la température voulue le liquide recyclé. A un niveau supérieur à celui de la conduite 16 se trouve une sortie 17, par laquelle le cyclohexane restant, le chlorure de cyclohexyle formé ainsi que le HCl vont dans un laveur 18, dans lequel l'eau introduite par 19 fait éliminer l'acide chlorhydrique sous la forme d'une solution aqueuse, évacuée en bas par 20. Le mélange cyclohexane/chlorure de cyclohexyle ainsi lavé passe par la canalisation 21 dans la colonne à distiller 22; le cyclohexane, distillé en haut de la colonne, retourne par 23 dans le réservoir de stockage 1, tandis que le chlorure de cyclohexyle brut passe, du bas de la colonne 22, par le conduit 24 dans une colonne de rectification classique, non représentée.

Sur la fig. 2, on voit le fond du réacteur photochimique 8 auquel est reliée la canalisation 7 au moyen d'un raccord 26. A l'entrée de ce dernier est insérée une grille ou plaque 28 en matériau inattaquable à l'acide chlorhydrique; à la sortie du raccord 26 se trouve une seconde grille ou plaque perforée similaire 28'. L'intérieur du raccord est garni de tournures ou de filaments 27 de métal inhibiteur de la réaction. Les grilles 28 et 28' sont calculées de façon à laisser passer librement le liquide venant de la canalisation 7, sans cependant permettre l'entraînement des tournures ou des filaments 27.

*Exemples 1 à 6*

Dans un appareil en verre, à l'échelle de laboratoire, conforme au schéma de la fig. 1, on a effectué une série d'essais de chloration de cyclohexane, avec ou sans garnissage de tournures de tantale à l'entrée du réacteur. Le volume du réacteur était de 160 ml, le débit de chlore de 11 à 12 l/h, mesuré à 0° C/760 mmHg.

La température dans le réacteur était maintenue à 22-23° C. Les temps de séjour dans le réacteur étaient réglés de façon que tout le chlore soit consommé avant la sortie du réacteur, alors que le débit de la circulation dans le circuit de recyclage 16-4-6 était modifié d'un essai à l'autre. La durée maximale de passage par le circuit de recyclage était de 0 h 32, l'appoint de cyclohexane frais étant de 500 ml/h. L'entrée du réacteur comportant un tube de 5 mm de diamètre intérieur dans lequel était placé, sur 2 cm de haut, un tampon de 2 g de tantale en tournures de 0,2 mm d'épaisseur; ce tampon était enlevé pour les essais des exemples 5 et 6. Le tableau I donne les résultats de ces essais.

La comparaison des productions et des sélectivités des exemples 5 et 6 avec celles des exemples 1 à 4 montre un avantage marqué de l'emploi d'un inhibiteur à l'entrée du réacteur; on voit en effet qu'avec ce dernier on atteint des sélectivités de 94,5 contre un maximum de 86,2 en l'absence d'inhibiteur.

Ces exemples prouvent, d'autre part, qu'il est possible de travailler avec des vitesses assez élevées à l'entrée du réacteur, conduisant à des temps de séjour dans ce dernier assez faibles.

*Exemples 7 à 11*

Dans l'appareil des exemples précédents, dont le réacteur photochimique à lampe coaxiale à UV centré sur 350 nm a une capacité de 160 ml, on produit du chlorure de cyclohexyle en continu; le cyclohexane est débité en un excès tel par rapport à $Cl_2$ que son taux de conversion en dérivé monochloré reste faible, dans les limites de 11 à 18%, le chlore étant complètement consommé dans le réacteur. On fait varier les débits des réactifs en valeur absolue, ainsi que la puissance de la lampe à UV. Dans les exemples 7 à 9, le garnissage de tantale est le même que dans les exemples 1 à 4, tandis que les exemples 10 et 11 sont effectués sans aucun inhibiteur.

Les résultats sont réunis dans le tableau II.

Il en résulte que, avec un garnissage de tantale, les sélectivités en produit désiré sont de 92 à 94% contre 81 à 84% en l'absence d'un tel inhibiteur; en outre, dans les exemples 10 et 11, du chlore est consommé dans la canalisation d'arrivée de mélange réactionnel, alors qu'il ne s'en consomme pas aux exemples 7 à 9.

*Tableau I*

| Exemple N° | Avec inhibiteur | | | | Sans inhibiteur | |
|---|---|---|---|---|---|---|
| | 1 | 2 | 3 | 4 | 5 | 6 |
| Débit de recyclage (l/h) | 5 | 35 | 70 | 150 | 5 | 150 |
| Vitesse linéaire à l'entrée du réacteur (m/s) | 0,07 | 0,5 | 1 | 2,13 | 0,07 | 2,13 |
| Mol/l $Cl_2$ à l'entrée du réacteur | 0,1 | 0,0143 | 0,00414 | 0,00373 | 0,1 | 0,00373 |
| Temps de séjour dans le réacteur (s) | 115,2 | 16,46 | 8,23 | 3,84 | 115,2 | 3,84 |
| Conversion du cyclohexane (%) | 11,24 | 11,35 | 13,80 | 11,43 | 11,24 | 11,43 |
| Production de monochlorocyclohexane (g/h) | 71,5 | 56,54 | 71,8 | 65,3 | 56,0 | 57,2 |
| Sélectivité en monochlorocyclohexane (mol-%) | 94,5 | 93,5 | 93,6 | 94,2 | 86,2 | 82,5 |

*Tableau II*

| Exemple N° | Avec inhibiteur | | | Sans inhibiteur | |
|---|---|---|---|---|---|
| | 7 | 8 | 9 | 10 | 11 |
| Débit de cyclohexane (ml/h) | 543 | 580 | 5000 | 543 | 5000 |
| Débit de $Cl_2$ gaz (l/h) | 11,5 | 21 | 130 | 11,5 | 130 |
| Rapport molaire cyclohexane/$Cl_2$ | 9,66 | 5,77 | 7,84 | 9,66 | 7,84 |
| Puissance électrique de la lampe (W) | 3,2 | 0,6 | 3,2 | 3,2 | 3,2 |
| Conversion du cyclohexane (%) | 11,4 | 18,5 | 13,2 | 11,6 | 13,3 |
| Production de monochlorocyclohexane (g/l/h) | 408 | 648 | 4493 | 361 | 3910 |
| Sélectivité en monochlorocyclohexane (%) | 94,2 | 92 | 94 | 83,4 | 81,8 |

## Revendications

1. Procédé d'halogénation d'un alcane ou d'un cycloalcane par l'action de la lumière ultraviolette sur une solution d'halogène dans l'alcane ou le cycloalcane, préparée d'avance qui passe dans la zone irradiée par l'ultraviolet, caractérisé en ce qu'un inhibiteur de l'halogénation est placé à l'entrée de la zone d'irradiation.

2. Procédé suivant la revendication 1, caractérisé en ce que l'inhibiteur de l'halogénation est également placé à la sortie de la zone d'irradiation.

3. Procédé suivant l'une des revendications 1 ou 2, caractérisé en ce que l'inhibiteur est un solide, insoluble dans le milieu réactionnel, en particulier un métal du groupe VB de la classification périodique des éléments.

4. Procédé suivant l'une des revendications précédentes, caractérisé en ce que l'inhibiteur de l'halogénation est constitué par un ou plusieurs des métaux suivants: vanadium, niobium et tantale.

5. Application du procédé suivant l'une des revendications 1 à 4 à la production de monochlorure de cyclohexyle par traitement du cyclohexane par du chlore, en milieu liquide, en présence de la lumière ultraviolette, le cyclohexane étant en excès par rapport au chlore, caractérisée en ce qu'un inhibiteur, constitué par du vanadium, du niobium ou du tantale, est placé à l'entrée de la zone de réaction photochimique, et de préférence également à la sortie de cette zone.

6. Application suivant la revendication 5, dans laquelle le milieu réactionnel est soumis à la recirculation par la zone soumise à l'ultraviolet et par un dispositif de refroidissement, caractérisée en ce qu'à l'entrée et à la sortie de la zone de réaction photochimique sont disposés des filaments ou des tournures de tantale présentant une surface de l'ordre de 100 à 10 000 cm² par centimètre carré de section de la veine liquide arrivant à la zone de réaction ou en partant.

7. Appareil pour la réalisation du procédé suivant l'une des revendications précédentes, qui comporte un réacteur (8) renfermant une lampe à ultraviolet (10), un mélangeur (4) recevant l'alcane ou le cycloalcane (3) et l'halogène (5), une pompe de circulation (6) alimentant le réacteur par une canalisation (7), une sortie (11) du réacteur vers un séparateur (14), un circuit de retour (16) du séparateur au mélangeur (4), muni d'un refroidisseur (25) et une conduite de départ (17) du séparateur vers un laveur (18) du produit de la réaction, caractérisé en ce que, à l'entrée (12) du réacteur, et de préférence également à la sortie (13) de celui-ci, sont disposés des raccords renfermant du vanadium, du niobium ou, plus particulièrement, du tantale à l'état divisé, notamment sous la forme de tournures ou de filaments.

## Patentansprüche

1. Verfahren zur Halogenierung eines Alkans oder eines Cycloalkans durch Einwirken von Ultraviolettlicht auf eine Halogenlösung in dem Alkan oder Cycloalkan, die vorher hergestellt wurde, die durch die ultraviolettbestrahlte Zone läuft, dadurch gekennzeichnet, dass am Eingang der Bestrahlungszone ein Halogenierungsinhibitor angebracht wird.

2. Verfahren nach Anspruch 1, dadurch gekennzeichnet, dass der Halogenierungsinhibitor auch am Ausgang der Bestrahlungszone angebracht wird.

3. Verfahren nach einem der Ansprüche 1 oder 2, dadurch gekennzeichnet, dass der Inhibitor ein in dem Reaktionsmedium unlöslicher Feststoff, insbesondere ein Metall der Gruppe VB des Periodensystems der Elemente ist.

4. Verfahren nach einem der vorhergehenden Ansprüche, dadurch gekennzeichnet, dass der Halogenierungsinhibitor aus einem oder mehreren der Metalle Vanadium, Niob und Tantal besteht.

5. Anwendung des Verfahrens gemäss einem der Ansprüche 1 bis 4 bei der Herstellung von Cyclohexylmonochlorid durch Behandeln von Cyclohexan mit Chlor in flüssigem Medium in Anwesenheit von Ultraviolettlicht, wobei das Cyclohexan im Überschuss, bezogen auf Chlor, vorliegt, dadurch gekennzeichnet, dass ein Inhibitor, der aus Vanadium, Niob oder Tantal besteht, am Eingang der photochemischen Reaktionszone und vorzugsweise auch am Ausgang dieser Zone angebracht wird.

6. Anwendung nach Anspruch 5, bei der das Reaktionsmedium durch die dem Ultraviolett ausgesetzte Zone und durch eine Abkühlvorrichtung rezirkuliert wird, dadurch gekennzeichnet, dass am Eingang und am Ausgang der photochemischen Reaktionszone Filamente oder Drehspäne aus Tantal angebracht sind, die eine Oberfläche in der Grössenordnung von 100 bis 10 000 cm² pro Quadratzentimeter des Schnitts der Flüssigkeitsader aufweisen, die an der Reaktionszone ankommt oder sie verlässt.

7. Vorrichtung zur Durchführung des Verfahrens gemäss einem der vorhergehenden Patentansprüche, enthaltend einen Reaktor (8), der eine Ultraviolettlampe (10), einen Mischer (4), der das Alkan oder Cycloalkan (3) und das Halogen (5) aufnimmt, eine Zirkulationspumpe (6), die den Reaktor über eine Leitung (7) bespeist, einen Ausgang (11) des Reaktors zu einem Separator (14) hin, eine Rückführleitung (16) vom Separator zum Mischer (4), ausgerüstet mit einem Kühler (25), und eine Ausgangsleitung (17) vom Separator zu einer Waschvorrichtung (18) für das Reaktionsprodukt umfasst, dadurch gekennzeichnet, dass am Eingang (12) des Reaktors und vorzugsweise auch an dessen Ausgang (13) Verbindungsstücke angebracht sind, die Vanadium, Niob oder insbesondere Tantal im verteilten Zustand, insbesondere in der Form von Drehspänen oder Filamenten, enthalten.

## Claims

1. Process of halogenation of an alkane or cycloalkane by the action of ultraviolet light upon a solution of the halogen in the alkane or cycloalkane prepared in advance which passes into a zone irradiated by ultraviolet, characterized in that a halogenation inhibitor is located at the inlet to the irradiation zone.

2. Process according to Claim 1, characterized in that the halogenation inhibitor is also located at the outlet from the irradiation zone.

3. Process according to Claim 1 or 2, characterized in that the inhibitor is a solid insoluble in the reaction medium, in particular a metal of Group VB of the Periodic Classification of the Elements.

4. Process according to any of the preceding claims, characterized in that the halogenation inhibitor comprises one or more of the metals vanadium, niobium and tantalum.

5. Application of the process according to any of Claims 1 to 4 to the production of cyclohexyl monochloride by the treatment of cyclohexane with chlorine in liquid medium in the presence of ultraviolet light, the cyclohexane being in excess with respect to the chlorine, characterized in that an inhibitor, comprising vanadium, niobium or tantalum, is located at the inlet to the photochemical reaction zone and preferably also at the outlet from this zone.

6. Application according to Claim 5, in which the reaction medium is subjected to recirculation through the zone subjected to the ultraviolet and through a cooling device, characterized in that, at the inlet and at the outlet of the photochemical reaction zone, filaments or turnings of tantalum are located, which have a surface area of the order of 100 to 10,000 cm² per centimetre of cross-section of the liquid stream entering or leaving the reaction zone.

7. Apparatus for carrying out the process according to any of the preceding claims, which comprises a reactor (8) containing an ultraviolet lamp (10), a mixer (4) receiving the alkane or cycloalkane (3) and the halogen (5), a circulation pump (6) supplying the reactor through a duct (7), an outlet (11) from the reactor to a separator (14), a return circuit (16) from the separator to the mixer (4) provided with a condenser (25) and an outlet conduit (17) from the separator to a scrubber (18) for the reaction product, characterized in that, at the inlet (12) to the reactor and preferably also at the outlet (13) therefrom, enclosures containing vanadium, niobium or more particularly tantalum in a sub-divided state are located, particularly in the form of turnings or filaments.

FIG_1

FIG_2

0 091 358